(19) Europäisches Patentamt
European Patent Office
Office européen des brevets

(11) Numéro de publication: **0 540 540 B1**

(12) **FASCICULE DE BREVET EUROPEEN**

(45) Date de publication de fascicule du brevet: **28.09.94**

(51) Int. Cl.[5]: **C01F 11/18**, G11B 5/72, C11D 9/12, A61K 7/16

(21) Numéro de dépôt: **91912420.6**

(22) Date de dépôt: **23.07.91**

(86) Numéro de dépôt internationale :
**PCT/BE91/00051**

(87) Numéro de publication internationale :
**WO 92/01629 (06.02.92 92/04)**

(54) **VATERITE ET SON PROCEDE DE FABRICATION.**

(30) Priorité: **24.07.90 BE 9000743**

(43) Date de publication de la demande:
**12.05.93 Bulletin 93/19**

(45) Mention de la délivrance du brevet:
**28.09.94 Bulletin 94/39**

(84) Etats contractants désignés:
**AT BE CH DE DK ES FR GB GR IT LI LU NL SE**

(56) Documents cités:
**EP-A- 140 644**
**EP-A- 197 327**
**DE-A- 3 619 909**
**FR-A- 2 405 903**
**US-A- 3 304 154**

**CHEMICAL ABSTRACTS, vol. 111, no. 26, 25. Dez. 1989, Columbus Ohio, US; abstract no. 236085T, K.TANAKA et al. voir abrégé & JP,A.62 113 718 (OKUTAMA KOGYO) 25 Mai 1987**

(73) Titulaire: **LHOIST RECHERCHE ET DEVELOPPEMENT S.A.**
**Rue Charles-Dubois, 28,**
**S.-Jean-des-Bois**
**B-1342 Ottignies-Louvain-La-Neuve (BE)**

(72) Inventeur: **GOFFIN, Robert**
**Avenue des Croisettes 34**
**B-4949 Trooz (BE)**
Inventeur: **LANGELIN, Henri-René**
**Rue de l'Eglise**
**F-60232 Caffiers (FR)**

(74) Mandataire: **Vanderperre, Robert et al**
**Bureau Vander Haeghen S.A.**
**Rue Colonel Bourg 108 A**
**B-1040 Bruxelles (BE)**

**EP 0 540 540 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

**PATENT ABSTRACTS OF JAPAN** vol. 11, no. 60 (C-405) 24 Fevr. 1987 & JP-A-61 219 716 (Agency of Industrial Science & Technology) 30 Sept. 1986

## Description

La présente invention a pour objet un procédé de fabrication de vatérite sensiblement sphérique, en particulier de vatérite de granulométrie déterminée.

On connaît par le document FR-A-2298511 un procédé de fabrication de vatérite. Dans ce procédé connu, on traite de l'oxyde de calcium dans un milieu réactionnel contenant des nitrates ou des chlorures et une amine, on élimine dudit milieu des insolubles et on introduit dans le milieu du $CO_2$, de manière à précipiter du carbonate de calcium sous forme d'aggregat de grains de vatérite. La vatérite ainsi produite n'est pas stable et ne permet pas non plus la production de dérivés calciques de très haute pureté, par exemple de pureté supérieure a 99,99%.

De plus, par ce procédé connu, on obtient un mélange de particules de vatérite dont le facteur granulométrique est supérieur a 1.

On connaît également par le document US-A-4871519 un procédé permettant de produire du carbonate de calcium de haute pureté (supérieure à 99%). Dans ce procédé, on traite de la dolomie décarbonatée dans une solution contenant un sel acide d'une amine organique de manière à transformer le calcium sous forme de sel soluble et après filtration on traite la solution au moyen de $CO_2$ de manière a précipiter du carbonate de calcium.

Tous les exemples de ce document ne permettent pas d'obtenir de la vatérite stable en particulier ayant un facteur granulométrique inférieur à 1.

On connaît encore par le document US-A-3,304,154 un procédé de préparation de carbonate de calcium.

Dans ce procédé, on prépare une suspension d'hydroxyde de calcium, on porte cette suspension à 60 °C et on la soumet à une agitation dans un autoclave.

Ensuite, du dioxyde de carbone est introduit dans la suspension jusqu'à obtention d'une pression de l'ordre de 4 à 6 bars (4 à 6 $10^5$ Pa) dans l'autoclave. La température de l'autoclave est ensuite maintenue à environ 75 °C de manière à éviter la formation de carbonate de calcium colloîdal et de manière à former des particules de forme sensiblement sphérique.

Ce procédé connu nécessite plusieurs étapes successives d'injection de $CO_2$ dans l'autoclave.

Ce procédé ne permet pas la préparation d'une vatérite sensiblement sphérique de granulométrie déterminée en particulier d'une vatérite présentant un facteur granulométrique inférieur à 0,5.

On connaît enfin par le document EP-A-0197 327 un procédé de fabrication de carbonate de calcium dans lequel on prépare une solution aqueuse d'hydroxyde de calcium contenant un ammonium ou une amine, on élimine des impuretés par filtration et charbon actif et on ajoute du $CO_2$ pour former du carbonate de calcium. Ce procédé ne permet pas d'obtenir une vatérite sensiblement sphérique de granulométrie déterminée en particulier d'une vatérite présentant un facteur granulométrique inférieur à 0,5.

Le facteur granulométrique est un paramètre permettant de déterminer la répartition granulométrique d'un mélange de particules. Un facteur granulométrique important signifie que le mélange contient une quantité importante de particules dont la granulométrie s'écarte de la granulométrie moyenne.

Le facteur granulométrique d'un mélange peut être déterminé par la formule suivante :

$$2 \times \frac{d_{80\%} - d_{20\%}}{d_{80\%} + d_{20\%}}$$

dans laquelle :

| | |
|---|---|
| $d_{80\%}$ | est le diamètre supérieur des particules de la fraction passante à 80% du mélange, |
| $d_{20\%}$ | est le diamètre supérieur des particules de la fraction passante à 20% du mélange, |
| "fraction passante à x%" | est la fraction contenant les particules du mélange ayant une granulométrie inférieure à une valeur déterminée, cette fraction correspondant à x% en poids du mélange. |

Un autre objet de la présente invention est une vatérite sous forme de particules dont le facteur granulométrique est inférieur à 0,5.

En particulier, elle a pour objet une vatérite de granulométrie inférieure à 20µ, avantageusement à 10µ. Dans une forme préférée, la vatérite selon l'invention a un diamètre moyen (moyenne en poids) inférieur à 5µ, avantageusement à 3µ, en particulier inférieur à 1µ.

Le procédé selon l'invention, en particulier le procédé de fabrication permettant d'obtenir une vatérite sensiblement sphérique, mon sous forme d'aggregat de grains, par exemple une vatérite sous forme de particules dont le facteur granulométrique est inférieur à 1, avantageusement à 0,5, comprend les étapes suivantes :

- on traite un mélange de particules contenant du calcium sous forme d'oxyde et/ou d'hydroxyde dans un milieu réactionnel contenant un sel acide d'une amine organique de manière à transformer le calcium sous forme de sel soluble et de manière à obtenir un milieu dont le pH dit pH de traitement, a une valeur supérieure à 10 ;
- on introduit du $CO_2$ dans le milieu soumis à une agitation jusqu'a ce que le pH du milieu atteigne un pH dit pH de saturation dont la valeur est proche de 0,83 x pH de traitement (de manière à saturer le milieu en $CO_2$), et
- on introduit du $CO_2$ à un débit inférieur à 50 g $CO_2$/heure/litre dans le milieu éventuellement soumis à une agitation de manière à former de la vatérite sensiblement sphérique, en particulier de granulométrie prédéterminée.

Selon une caractéristique de ce procédé, on introduit du $CO_2$ dans le milieu lorsque la teneur en calcium du milieu est comprise entre 0,3 et 1, avantageusement de 0,35 à 0,65, de préférence entre 0,45 et 0,55 mole/litre.

Eventuellement, le procédé peut comporter une étape dans laquelle on ajuste la teneur en Ca avant l'introduction du $CO_2$ dans le milieu.

Selon une autre caractéristique, on traite un mélange de particules contenant du CaO dans le milieu réactionnel à une température inférieure à 50-60 °C, de préférence inférieure à 20 °C.

De façon avantageuse on introduit du $CO_2$ dans le milieu lorsque la température du milieu est inférieure à 40 °C, de préférence à 20 °C. Dans une forme de réalisation, forme préférée lorsque le mélange de particules contenant du calcium contient également des impuretés telles que des dérivés de magnésium (MgO, $Mg(OH)_2$), on élimine des insolubles du milieu après le traitement du mélange dans le milieu réactionnel. Cette élimination peut être réalisée par filtration, décantation, etc.

Pour favoriser cette élimination des impuretés, le demandeur a remarqué qu'il était utile de traiter le mélange de particules contenant du calcium dans un milieu réactionnel contenant un sel acide d'une amine organique en présence de silice et eventuellement d'un additif tel qu'un support poreux, des zéolithes, l'alumine, du fer ou des dérivés du fer.

Le rapport en poids $SiO_2$/CaO ou $Ca(OH)_2$ est compris entre 0,5 et 10, tandis que le rapport en poids de fer et/ou d'alumine/CaO ou $Ca(OH)_2$ est avantageusement inférieur à 0,1, de préférence compris entre 0,005 et 0,03. Dans une forme de réalisation préférée on introduit du $CO_2$ dans le milieu en trois étapes, à savoir :

- l'introduction du $CO_2$ jusqu'à l'abaissement du pH à un pH dit de saturation dont la valeur est voisine de 0,83 x pH de traitement ;
- l'introduction de $CO_2$ à un débit inférieur à 50, avantageusement à 20g $CO_2$/heure/litre jusqu'à l'abaissement du pH à un pH dit de nucléation dont la valeur est proche de 0,8 x pH de traitement, et
- l'introduction du $CO_2$ à un débit inférieur à 50 avantageusement à 20 g $CO_2$/heure/litre pour abaisser le pH à un pH de cristallisation dont la valeur est inférieure à 0,75 x pH de traitement, de préférence comprise entre 0,7 et 0,75 x pH de traitement.

Le pH dit de nucléation correspond avantageusement à environ 0,97 x pH de saturation tandis que le pH dit de cristallisation est avantageusement inférieur à 0,93 x pH de nucléation.

De façon avantageuse, on arrête l'admission de $CO_2$ dans le milieu entre lesdites trois étapes.

Selon une particularité de cette forme de réalisation, on met le milieu réactionnel sous une pression supérieure à 2 $10^5$ Pa lors de la première étape d'introduction de $CO_2$ dans le milieu. Lors de cette première étape, le débit de $CO_2$ introduit dans le milieu est avantageusement supérieur à 50g $CO_2$/heure/litre.

Selon d'autres particularités du procédé suivant l'invention, on soumet le milieu à une agitation vigoureuse lors de la première étape d'introduction de $CO_2$ et à une agitation modérée lors des deuxième et troisième étapes d'introduction de $CO_2$.

Par agitation vigoureuse, on entend une agitation se caractérisant par un nombre de Reynold supérieur à 8000, tandis que par agitation modérée, on enterid une agitation se caractérisant par un nombre de

Reynolds inférieur à 5000, de préférence supérieur à 10.

Ce nombre de Reynolds est estimé par la formule suivante :

$$Re = D_a^2 N \cdot \rho / \mu$$

dans laquelle

Re nombre de Reynolds

$D_a$ diamètre des pales de l'agitateur (m)

$\rho$ densité du milieu ($kg/m^3$)

$\mu$ viscosité ($Pa\ s^{-1}$).

Un nombre de Reynolds supérieur à 10000 correspond à un mouvement turbulent dans le milieu, tandis qu'un nombre de Reynolds inférieur à 10 correspond à un mouvement laminaire.

Le sel acide d'une amine organique est de préférence un composé de formule générale 1 :

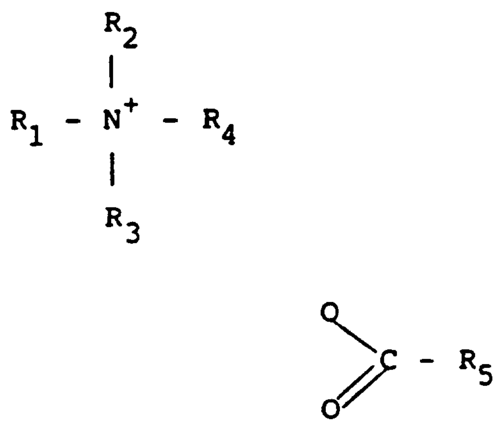

dans laquelle :

au moins $R_1$ ou $R_2$ ou $R_3$ ou $R_4$ est un groupe hydrocarboné avantageusement substitué par au moins un groupe hydroxyle.

De préférence, les radicaux $R_1$, $R_2$, $R_3$, $R_4$ et $R_5$ ont les significations suivantes :

$R_1$ est un radical hydrocarboné substitué par au moins un groupe hydroxyle, ce radical ayant de 1 à 10 atomes de carbone ;

$R_2$, $R_3$ et $R_4$ sont un atome d'hydrogène ou un radical hydrocarboné éventuellement substitué par un ou plusieurs groupes hydroxyles, ce radical hydrocarboné ayant avantageusement de 1 à 10 atomes de carbone, et

$R_5$ est un atome d'hydrogène ou un radical hydrocarboné en $C_1$-$C_{10}$ éventuellement substitué par un ou plusieurs groupes hydroxyles ou carboxyles.

De préférence $R_1$ est un groupe linéaire ou branché mais substitué par au moins un groupe hydroxyle. A titre d'exemple, $R_1$ peut être choisi parmi : l'éthyle, l'hydroxyméthyle, le 2-hydroxyéthyle, le 1-hydroxyéthyle, le 3-hydroxypropyle, le 2-hydroxypropyle, le 1-hydroxyisopropyle, le 4-hydroxybutyle, le 3-hydroxybutyle, le 2-hydroxyisobutyle, le 3-hydroxy-2-méthylpropyle, le 5-hydroxypentyle, le 3-hydroxyméthylbutyle, le 2-hydroxyisopentyle, le 3-hydroxy-1,1-diméthylpropyle, le 3-hydroxy 1,2-diméthylpropyle.

$R_2$, $R_3$ et $R_4$ sont avantageusement un atome d'hydrogène, un groupe méthyle, éthyle, hydroxyéthyle, propyle ou hydroxypropyle.

$R_5$ est avantageusement un atome d'hydrogène ou un radical en $C_1$-$C_5$. De préférence, le groupe $^{-}OOC$-$R_5$ est le reste d'un acide choisi parmi l'acide formique, l'acide acétique, l'acide citrique, l'acide propanoïque, l'acide pentanoïque, l'acide méthylpropanoïque, l'acide diméthylpropanoïque, l'acide méthylbutanoïque, l'acide oxopentanoïque ou un mélange de ceux-ci.

Comme exemple de composé de formule 1 on peut citer l'acétate ou le formiate de diéthylamine, l'acétate ou le formiate de monoéthanolamine.

Si y est le nombre de fonctions acides du composé, le rapport molaire oxyde de calcium/composé de formule 1 est supérieur à 0,1 et est, de préférence, compris entre 0,5 y et 1 y. De façon avantageuse, ce

rapport molaire est voisin de 0,6 y.

D'autres particularités et détails de l'invention ressortiront de la description détaillée suivante dans laquelle il est fait référence à des exemples de préparation ainsi qu'aux dessins ci-annexés.

Dans ces dessins,

- les figures 1 à 3 montrent la répartition granulométrique de particules de vatérite obtenues par un procédé suivant l'invention, (fraction passante exprimée en % en poids en fonction de la granulométrie exprimée en $\mu$), et
- la figure 4 est une vue en coupe à plus grande échelle d'un grain de vatérite obtenu par un procédé suivant l'invention.

Exemple 1

On a préparé un milieu réactionnel aqueux en mélangeant un formiate d'éthanolamine (un ester d'aminoalcool de formule $CH_2OH\ CH_2\ NH_3\ COOH$) de manière à obtenir un milieu réactionnel avec une concentration de 1 mole d'ester par litre.

On a ensuite ajouté audit milieu réactionnel de la chaux CaO sous forme de poudre à raison de 0,5 mole par litre de milieu réactionnel (rapport molaire CaO/ester : 0,5).

La temperature du milieu réactionnel a été maintenue lors de ce traitement rapide de la chaux à environ 20 ° C.

La réaction suivante a eu lieu : $CaO + 2\ CH_2OH.CH_2.NH_3^+$,

$$HCOO^- \xrightarrow{H_2O} Ca^{++},$$

$2(HCOO)^- + 2CH_2OH\ CH_2NH_3^+,\ OH^-$.

Le pH du milieu réactionnel est passé, lors de la réaction avec la choux, d'environ 6 à environ 11,5 - 12.

On a ensuite filtré au moyen d'un filtre a précouche le milieu réactionnel de manière à éliminer les impuretés métalliques qui, à un tel pH, précipitent. On a ainsi pu éliminer toutes traces d'aluminium, de fer et de métaux lourds.

Après filtration le milieu réactionnel dont la concentration en Ca était voisine de 0,5 a été soumis à un barbotage de $CO_2$ (température du milieu : 20 ° C).

Ce barbotage a été effectué de la manière suivante :

Dans un premier temps, on a introduit du $CO_2$ gazeux dans le milieu à un débit de 70g $CO_2$/heure/litre de solution de manière à abaisser le pH du milieu jusqu'a une valeur d'environ 9,8. Cette opération a été effectuée dans une cuve maintenue sous une pression de 2 à 3 $10^5$ Pa et soumise à l'action d'un agitateur à pales du type à ancre. Cet agitateur avait un diamètre de 60mm tandis que sa vitesse de rotation était de 500-600 tours/minute. La cuve cylindrique avait un diamètre de 200mm. (volume du réacteur 2 litres).

Le mouvement créé par l'agitateur dans la cuve était sensiblement turbulent.

Lorsque le pH du milieu était proche de 9,8, on a arrêté l'alimentation en $CO_2$ et le milieu n'a plus été maintenu sous pression, ni soumis à l'action de l'agitateur pendant quelques minutes.

On a ensuite introduit du $CO_2$ gazeux dans le milieu réactionnel à un débit de 10-15g $CO_2$/heure/litre pour abaisser le pH du milieu jusqu'à 9,5 -9,6. La cuve contenant le milieu réactionnel n'était pas mise sous pression. Lors de cette introduction de $CO_2$ le milieu réactionnel était soumis à une agitation modérée, la vitesse de rotation des pales étant de 100 tours/minute.

Lorsque le pH du milieu était voisin de 9,5-9,6, on a arrêté l'alimentation en $CO_2$ pendant quelques minutes, tout en maintenant le milieu sous agitation modérée (vitesse de rotation : 100 tours/minute).

Enfin, on a introduit du $CO_2$ à un débit de 10-15g $CO_2$/heure/litre de solution dans la solution maintenue sous agitation modérée (vitesse de rotation : 100 tours/minute) jusqu'à l'abaissement du pH du milieu à une valeur d'environ 8,5.

On a ainsi précipité de la vatérite que l'on a récupérée par filtration et que l'on a lavée au moyen d'une solution 50% en poids eau, 50% en poids méthanol.

La distribution granulométrique de la vatérite ainsi produite est représentée à la figure 1.

Cette vatérite avait les caractéristiques suivantes :

- pureté supérieure à 99,99%
- surface spécifique 30 $m^2$/g
- stabilité dans le milieu réactionnel supérieure à 24 heures

- granulométrie moyenne : 1μ
- facteur granulométrique :

$$2x \frac{(1,3 - 0,9)}{(1,3 + 0,9)} = 0,36$$

Grâce au barbotage de $CO_2$, on a reformé le milieu réactionnel de départ de sorte que ce dernier peut être utilisé pour traiter une nouvelle charge de CaO.

Exemples 2 et 3

On a préparé de la vatérite de la manière décrite dans l'exemple 1 si ce n'est que dans les deuxième et troisième phases d'introduction de $CO_2$ dans le milieu la vitesse de rotation de l'agitateur était respectivement de 125 et 150 tours/minute.

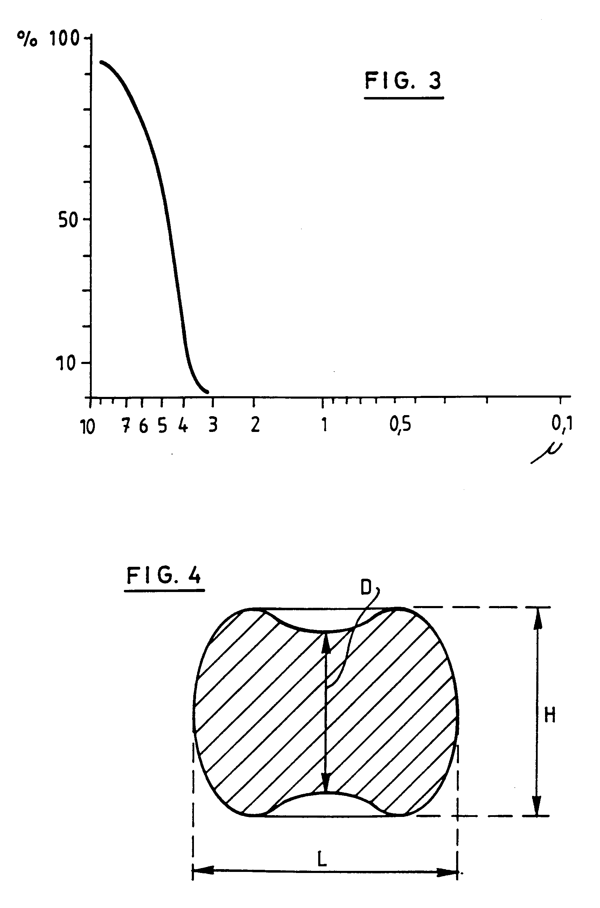

La répartition granulométrique de la vatérite ainsi produite est représentée à la figure 2 pour une vitesse de rotation de 125 tours/minute lors de la deuxième et troisième phases d'introduction de $CO_2$ dans le milieu et à la figure 3 pour une vitesse de rotation de 150 tours/minute lors des deuxième et troisième phases d'introduction de $CO_2$ dans le milieu.

Le tableau suivant reprend les valeurs caractérisant la vatérite produite dans les exemples 1 à 3.

TABLEAU

| Vitesse de rotation lors des 2ème et 3ème phases | diamètre moyen μ | facteur de répartition granulométrique |
|---|---|---|
| 100 | 1 | 0,36 |
| 125 | 3 | 0,46 |
| 150 | 5 | 0,43 |

Ce tableau montre qu'il est possible grâce au procédé suivant l'invention de produire de la vatérite présentant une granulométrie moyenne prédéterminée, telle qu'une vatérite présentant un facteur de répartition granulométrique inférieur à 0,5 en sélectionnant une vitesse de rotation de l'agitateur, c'est-à-dire en choisissant un mouvement pouvant se caractériser par un nombre de Reynolds déterminé.

On a calciné la vatérite de l'exemple 1 dans un four à une température de 1100 °C de manière à obtenir, comme dérivé calcique, de la chaux extra pure CaO.

Cet oxyde de calcium extra pur avait les propriétés suivantes :

- pureté : + de 99,9%
- granulométrie :<20 microns
- Aluminium : aucune trace détectée
- Surface BET : 20 $m^2$/g.

Lors de la calcination, des acides organiques ou sels de ceux-ci encore présents, sous forme de trace, dans la vatérite sont décomposés en $H_2O$, $CO_2$, c'est-à-dire en produits qui ne provoquent pas de pollution de l'oxyde de calcium.

Exemple 4

On a répété la méthode suivie dans l'exemple 1 pour la préparation de carbonate de calcium et d'oxyde de calcium, si ce n'est

- que l'on a utilisé de l'acétate d'éthanolamine au lieu de formiate d'éthanolamine,
- que la température du milieu lors du traitement de la chaux contenant un peu de dolomie était de 40 °C au lieu de 20 °C, et
- que le barbotage de $CO_2$ dans le milieu filtré a été effectué dans une enceinte sous pression (2 $10^5$ Pascal)

La vatérite obtenue par le procédé utilisant de l'acétate d'éthanolamine avait les propriétés suivantes :

- pureté : + de 99,99% de $CaCO_3$
- Aluminium : aucune trace détectée
- Fer : aucune trace détectée
- Métaux lourds : aucune trace détectée
- Surface spécifique : 25 $m^2$/g.

Exemples 5 à 8

On a répété l'exemple 1 si ce n'est que l'on a fait varier la concentration en acétate de calcium du milieu. Le tableau suivant reprend les résultats de ces essais.

| Exemple | Concentration en Ca (mole/litre) | Remarques |
|---|---|---|
| 5 (exemple comparatif) | 0,75 | formation de vatérite ainsi que d'agglomérat de grains de vatérite |
| 6 | 0,5 | formation de vatérite sphérique |
| 7 | 0,45 | formation de vatérite sphérique |
| 8 (exemple comparatif) | 0,3 | formation de cristaux dont la forme se rapproche de celle de lentilles |

Ce tableau montre que le choix judicieux de la concentration en Ca dans le milieu réactionnel permet d'obtenir par le procédé suivant l'invention de la vatérite sensiblement sphérique. Ainsi dans le procédé suivant l'invention, la concentration en Ca du milieu est avantageusement comprise entre 0,35 et 0,65, de préférence entre 0,45 et 0,5 mole/litre.

La vatérite produite dans les exemples 6 et 7 se présentait sous la forme d'une sphère présentant deux creux diamétralement opposés, la hauteur H de la vatérite dans le sens du diamètre D reliant les deux creux correspondant à environ 70% de la largeur L de la vatérite mesurée dans un plan perpendiculaire audit diamètre. Une telle vatérite est représentée en coupe à la figure 4.

La stabilité de la vatérite suivant l'invention a été comparée à celle de la vatérite obtenue par un procédé connu. Cette étude de la stabilité a été effectuée au moyen d'un microscope électronique à balayage (T 330 A - Japanese Electronical Optical Limited).

Les résultats de cette étude sont donnés dans le tableau suivant :

TABLEAU

| | Vatérite suivant l'invention | Vatérite obtenue par un procédé connu |
|---|---|---|
| après 10' | vatérite | vatérite |
| après 15' | vatérite | transformation en calcite |
| après 24 heures | vatérite | |

Exemple 9

On a répété la méthode suivie dans l'exemple 1 pour la préparation de carbonate de calcium si ce n'est que l'on a ajouté au mélange de particules de chaux CaO des particules de silice, de fer et d'alumine. Le rapport en poids $SiO_2$/CaO était de 0,5, tandis que le % de fer et d'alumine était d'1 % par rapport au poids de CaO.

On a remarqué que l'opération de filtration du milieu réactionnel était ainsi améliorée, de sorte que la séparation des impuretés aurait pu être réalisée par simple décantation. L'utilisation de tels additifs permettait d'obtenir par décantation après 2 heures une solution limpide. A titre de comparaison, en n'utilisant aucun desdits additifs et par décantation, il n'était pas possible d'obtenir après 24 heures une solution limpide.

La vatérite selon l'invention peut être utilisée pour la preparation de :

- un mélange de vatérite suivant l'invention. (Un tel mélange peut se caractériser par la présence de plusieurs vatérites selon l'invention de facteur granulométrique inférieur à 1, de préférence à 0,5) ;
- une vatérite suivant l'invention munie d'une couche d'un métal choisi parmi les métaux précieux, en particulier l'or, l'argent et le platine ;
- des bandes magnétiques comprenant par exemple 4% de particules de vatérite munie d'une couche métallisée ;
- un savon, détergent, une pate dentifrice ou un produit cosmétique contenant une vatérite suivant l'invention ;
- un papier contenant une vatérite suivant l'invention ;
- une colle contenant une vatérite suivant l'invention ;
- une peinture contenant une vatérite suivant l'invention ;
- une sauce de couchage pour papier, ladite sauce contenant une vatérite suivant l'invention ;
- un lubrifiant à base d'une vatérite suivant l'invention ;
- un agent thixotrope (pour savon) à base d'une vatérite suivant l'invention ;
- un agent de traitement de surface à base d'une vatérite suivant l'invention ;
- une poudre de colmatage par exemple pour assurer une étanchéité aux raccords d'une canalisation d'eau, ladite poudre contenant une vatérite suivant l'invention.

La vatérite suivant l'invention grâce à sa forme et grâce à son facteur granulométrique permet d'obtenir une bonne adhérence des particules avec la colle ou la peinture, mais permet surtout d'éviter d'obtenir une couche non régulière de la colle ou peinture. Elle permet aussi d'éviter dans le cas des pâtes dentifrices, la formation de griffes sur les dents mais favorise surtout l'élimination des déchets, etc présents sur ou entre les dents. Une telle élimination est réalisée à la fois lors du brossage des dents mais aussi lors du rinçage.

Il est évident que si l'on peut obtenir un carbonate de calcium extra pur il est également possible d'obtenir des dérivés calciques extra purs. A titre d'exemple uniquement, on citera comme dérivés calciques, obtenus par transformation du carbonate de calcium obtenu par le procédé suivant l'invention l'oxyde de calcium, l'hydroxyde de calcium, le chlorure de calcium, le carbonate de calcium, le fluorure de calcium, l'oxalate de calcium, le citrate de calcium, les phosphates de calcium, ainsi que tous les sels de calcium insolubles dérivant d'un acide minéral et/ou organique.

Les dérivés calciques de haute pureté suivant l'invention peuvent être utilisés dans la préparation de compositions alimentaires (utilisation de citrate de calcium dans des laits pour bébé) ou de compositions pharmaceutiques, la fabrication de bandes magnétiques, etc.

La présente invention a encore pour objet un mélange de particules contenant du calcium sous forme d'oxyde et/ou d'hydroxyde convenant pour la mise en oeuvre d'un procédé suivant l'invention. Ce mélange comprend au moins un additif, par exemple

- de 0,5 à 10, de préférence de 0,5 à 1 part en poids de silice par part en poids de CaO et/ou $Ca(OH)_2$, et eventuellement
- moins de 0,1 peut en poids, de preference de 0,01 à 0,03 peut en poids d'alumine et/ou de zeolithe et/ou de fer (ou dérivés du fer) et/ou autres supports poreux par part en poids de CaO et/ou $Ca(OH)_2$.

## Revendications

**1.** Procédé de fabrication de vatérite sphérique, dans lequel

on traite un mélange de particules contenant du calcium sous forme d'oxyde et/ou d'hydroxyde dans un milieu réactionnel contenant un sel acide d'une amine organique de manière à transformer le calcium sous forme de sel soluble et à obtenir un milieu dont le pH dit pH de traitement a une valeur supérieure à 10 et dont la teneur en calcium est comprise entre 0,35 et 0,65 mole/litre,

on introduit du $CO_2$ dans le milieu soumis à une agitation jusqu'à ce que le pH du milieu atteigne un pH dit de saturation dont la valeur est proche de 0,83 x pH de traitement, et

on introduit du $CO_2$ à un débit inférieur à 50 g $CO_2$/heure/litre dans le milieu de manière à former de la vatérite sensiblement sphérique.

**2.** Procédé suivant la revendication 1, caractérisé en ce que, après avoir amené le pH du milieu au pH de saturation, on introduit du $CO_2$ à un débit inférieur à 50 g $CO_2$/heure/litre dans le milieu de manière à former de la vatérite sensiblement sphérique de granulométrie déterminée.

**3.** Procédé suivant la revendication 1 ou 2, caractérisé en ce qu'on introduit du $CO_2$ dans le milieu lorsque la teneur en calcium du milieu est comprise entre 0,45 et 0,55 mole/litre.

**4.** Procédé suivant l'une des revendications 1 à 3, caractérisé en ce qu'on ajuste la teneur en calcium du milieu de manière à ce qu'elle soit comprise entre 0,35 et 0,65, de préférence entre 0,45 et 0,55 mole/litre avant d'introduire dans le milieu du $CO_2$.

**5.** Procédé suivant l'une quelconque des revendications 1 à 4, caractérisé en ce qu'on introduit du $CO_2$ dans le milieu lorsque la température du milieu est inférieure à 40 ° C, de préférence à 20 ° C.

**6.** Procédé suivant l'une quelconque des revendications précédentes, caractérisé en ce qu'on introduit du $CO_2$ dans le milieu en trois étapes, à savoir :
- introduction de $CO_2$ jusqu'à l'abaissement du pH à un pH dit de saturation dont la valeur est voisine de 0,83 x pH de traitement ;
- introduction de $CO_2$ à un débit inférieur à 50 g $CO_2$/heure/litre jusqu'à l'abaissement du pH à un pH dit de nucléation dont la valeur est voisine de 0,8 x pH de traitement, et
- introduction de $CO_2$ à un débit inférieur à 50 g $CO_2$/heure/litre jusqu'à l'abaissement du pH à un pH dit de cristallisation dont la valeur est inférieure à 0,75 x pH de traitement,

ces trois étapes étant séparées entre elles par une étape intermédiaire dans laquelle on n'introduit pas de $CO_2$ dans le milieu.

**7.** Procédé suivant l'une quelconque des revendications 1 à 6, caractérisé en ce qu'on traite le mélange de particules contenant du calcium à une température inférieure à 50 ° C, de préférence comprise entre 20 et 30 ° C.

**8.** Procédé suivant l'une quelconque des revendications précédentes, caractérisé en ce qu'on met le milieu réactionnel sous une pression supérieure à 2 $10^5$ Pa lors de la première étape d'introduction de $CO_2$ dans le milieu.

**9.** Procédé suivant l'une quelconque des revendications précédentes, caractérisé en ce qu'on soumet le milieu à une agitation vigoureuse (nombre de Reynolds $N_{Re} \geqq 8000$) lors de la première étape d'introduction de $CO_2$ dans le milieu.

**10.** Procédé suivant l'une quelconque des revendications précédentes, caractérisé en ce qu'on soumet le milieu à une agitation modérée ($N_{Re} \leqq 5000$) lors de la deuxième et éventuellement de la troisième étape d'introduction de $CO_2$ dans le milieu.

**11.** Procédé suivant l'une quelconque des revendications précédentes, caractérisé en ce que le sel acide d'amine organique est un composé de formule générale 1 :

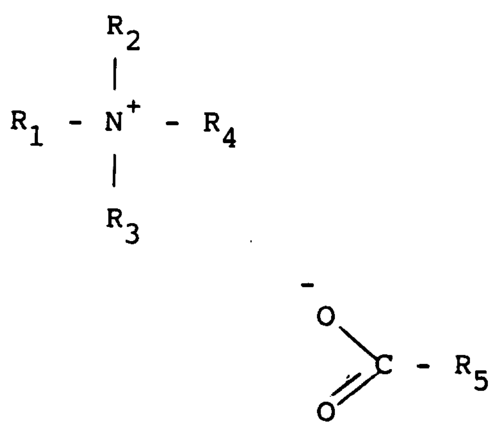

(1)

dans laquelle :
au moins $R_1$ ou $R_2$ ou $R_3$ ou $R_4$ est un groupe hydrocarboné avantageusement substitué par au moins un groupe hydroxyle.

**12.** Procédé suivant la revendication 11, caractérisé en ce que le sel acide d'amine organique est un composé de formule 1 dans laquelle les radicaux $R_1$, $R_2$, $R_3$, $R_4$ et $R_5$ ont les significations suivantes :
$R_1$ est un radical hydrocarboné substitué par au moins un groupe hydroxyle, ce radical ayant de 1 à 10 atomes de carbone ;
$R_2$, $R_3$ et $R_4$ sont un atome d'hydrogène ou un radical hydrocarboné éventuellement substitué par un ou plusieurs groupes hydroxyles, ce radical hydrocarboné ayant avantageusement de 1 à 10 atomes de carbone, et
$R_5$ est un atome d'hydrogène ou un radical hydrocarboné en $C_1$-$C_{10}$ éventuellement substitué par un ou plusieurs groupes hydroxyles ou carboxyles.

**13.** Procédé suivant la revendication 12, caractérisé en ce que le sel acide de formule 1 est choisi parmi le groupe comprenant l'acétate ou le formiate de diéthylamine, l'acétate ou le formiate de monoéthanolamine.

**14.** Procédé suivant l'une quelconque des revendications précédentes, caractérisé en ce qu'on élimine des insolubles du milieu après le traitement du mélange de particules avec un sel acide d'amine organique.

**15.** Procédé suivant la revendication 14, caractérisé en ce qu'on traite le mélange de particules contenant du calcium dans un milieu réactionnel contenant un sel acide d'une amine organique en présence d'un additif choisi parmi la silice, l'alumine, le fer ou dérivé du fer, les zéolithes et tout autre support poreux.

**16.** Vatérite sensiblement sphérique, susceptible d'être obtenue par un procédé suivant l'une quelconque des revendications 1 à 15, caractérisée en ce qu'elle se présente sous la forme d'une sphère présentant deux creux diamétralement opposés, et en ce qu'elle présente un facteur granulométrique inférieur à 0,5,
le facteur granulométrique étant déterminé par la formule suivante :

$$2 \times \frac{d_{80\,\%} - d_{20\,\%}}{d_{80\,\%} + d_{20\,\%}}$$

dans laquelle :

| | |
|---|---|
| $d_{80\,\%}$ | est le diamètre supérieur des particules de la fraction passante à 80 % du mélange, |
| $d_{20\,\%}$ | est le diamètre supérieur des particules de la fraction passante à 20 % du mélange, |
| "fraction passante à x %" | est la fraction contenant les particules du mélange ayant une granulométrie inférieure à une valeur déterminée, cette fraction correspondant à x % en poids du mélange. |

**17.** Vatérite suivant la revendication 16, caractérisée en ce qu'elle a une granulométrie inférieure à 20 $\mu$, de préférence à 5 $\mu$.

**18.** Vatérite suivant la revendication 17, caractérisée en ce qu'elle a une granulométrie moyenne inférieure ou égale à 5 $\mu$.

**19.** Vatérite suivant la revendication 18, caractérisée en ce qu'elle a une granulométrie moyenne inférieure ou égale à 3 $\mu$, de préférence à 1 $\mu$.

**20.** Vatérite suivant l'une quelconque des revendications 15 à 19, caractérisée en ce qu'elle est munie d'une couche d'un métal choisi parmi les métaux précieux.

## Claims

1. Process for the preparation of spherical vaterite in which
a mixture of particles containing calcium in the form of oxide and/or hydroxide is treated in a reaction medium containing an acid salt of an organic amine so as to convert the calcium into soluble salt and so as to obtain a medium, the pH of which -called pH of treatment- has a value higher than 10, while the calcium content of which is comprised between 0.35 and 0.65 mole/litre ;
$CO_2$ is introduced into the medium submitted to a stirring so that the pH of the medium reaches a pH -called pH of saturation-, the value of which is about 0.83 x pH of treatment, and
$CO_2$ is introduced in the medium at a rate lower to 50g $CO_2$/hour/litre so as to form substantially spherical vaterite.

2. Process according to claim 1, characterized in that after adjusting the pH of the medium to the pH of saturation $CO_2$ is introduced in the medium at a rate lower to 50g $CO_2$/hour/litre so as to form substantially spherical vaterite with a determined granulometry.

3. Process according to claim 1 or 2, characterized in that $CO_2$ is introduced in the medium when the calcium content of the medium is comprised between 0.45 and 0.55 mole/liter.

4. Process according to anyone of claims 1 to 3, characterized in that the calcium content of the medium is adjusted so that said content is comprised between 0.35 and 0.65, preferably between 0.45 and 0.55 mole/litre before introducing $CO_2$ into the medium.

5. Process according to anyone of claims 1 to 4, characterized in that $CO_2$ is introduced in the medium when the temperature of the medium is lower than 40 °C, preferably than 20 °C.

6. Process according to anyone of the preceding claims, characterized in that $CO_2$ is introduced into the medium in three steps, namely :
   - feeding $CO_2$ so as to lower the pH to a pH called pH of saturation, the value of which is about 0.83 x pH of treatment ;
   - feeding $CO_2$ at a rate less than 50g $CO_2$/hour/liter so as to lower the pH to a pH called pH of nucleation the value of which is about 0.8 x pH of treatment, and
   - feeding $CO_2$ at a rate lower than 50g $CO_2$/hour/liter up to the lowering of the pH to a pH called pH of cristallization, the value of which is less than 0.75 x pH of treatment, preferably comprised between 0.7 and 0.75 x pH of treatment

   these three steps being separated each from the other by an intermediate step in which no $CO_2$ is introduced into the medium.

7. Process according to anyone of the claims 1 to 6, characterized in that the mixture of particles containing calcium is treated at a temperature lower than 50 °C, preferably comprised between 20 and 30 °C.

8. Process according to anyone of the preceding claims, characterized in that the reaction medium is submitted to a pressure higher than 2 $10^5$ Pa for the first step of feeding $CO_2$ into the medium.

9. Process according to anyone of the preceding claims, characterized in that the medium is submitted to a vigorous stirring (Reynolds number $N_{Re} \geqq 8000$) for the first step of feeding $CO_2$ into the medium.

10. Process according to anyone of the preceding claims, characterized in that the medium is submitted to a moderate stirring ($N_{Re} \leqq 5000$) for the second and possibly third steps of feeding $CO_2$ into the medium.

11. Process according to anyone of the preceding claims, characterized in that the acid salt of organic amine is a compound of general formula 1

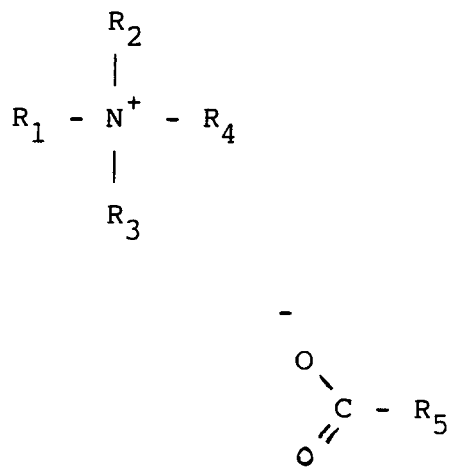

(1)

in which
at least $R_1$ or $R_2$ or $R_3$ or $R_4$ is a hydrocarbon group which is advantageously substituted by at least one hydroxyl group.

**12.** Process according to claim 11, characterized in that the acid salt of organic amine is a compound of formula 1 in which the radicals $R_1$, $R_2$, $R_3$, $R_4$ and $R_5$ have the following meanings :
$R_1$ is a hydrocarbon radical substituted by at least one hydroxyl group, this radical having 1 to 10 carbon atoms ;
$R_2$, $R_3$ and $R_4$ are a hydrogen atom or a hydrocarbon radical possibly substituted by one or more hydroxyl groups, the hydrocarbon radical having advantageously 1 to 10 carbon atoms, and
$R_5$ is a hydrogen atom or a $C_1$-$C_{10}$ hydrocarbon radical possibly substituted by one or more hydroxyl or carboxyl groups.

**13.** Process according to claim 12, characterized in that the acid salt of formula 1 is selected from the group consisting of the diethylamino acetate or formate and the monoethanolamino acetate or formate.

**14.** Process according to anyone of the preceding claims, characterized in that insolubles are removed from the medium after the treatment of the mixture of particles with an acid salt of an organic amine.

**15.** Process according to claim 14, characterized in that the mixture of particles containing calcium is treated in the reaction medium containing an acid salt of an organic amine in presence of an additive selected among silica, alumina, iron or iron derivative, zeolithe and any other porous carrier.

**16.** Substantially spherical vaterite susceptible to be obtained by a process according to anyone of the claims 1 to 15, characterized in that it has the shape of a sphere with two diametrically opposite hollows and in that it has a grain size distribution factor lower to 0.5, the grain size distribution factor being calculated by the following formula :

$$2 \times \frac{d_{80\%} - d_{20\%}}{d_{80\%} + d_{20\%}}$$

in which

| | |
|---|---|
| $d_{80\%}$ is | the upper diameter of the particles of the fraction of the mixture passing at 80% |
| $d_{20\%}$ is | the upper diameter of the particles of the fraction of the mixture passing at 20% |
| fraction passing at x% | is the fraction containing the particles of the mixture having a size lower |

than a determined value, this fraction corresponding to x% by weight of the mixture.

**17.** Vaterite according to claim 16, characterized in that it has a size lower than 20μ, preferably than 5μ.

**18.** Vaterite according to claim 17, characterized in that it has a mean size lower than or equal to 5μ.

**19.** Vaterite according to claim 18, characterized in that it has a mean size lower than or equal to 3μ, preferably 1μ.

**20.** Vaterite according to anyone of the claims 16 to 19, characterized in that it is provided with a layer of a metal selected among the precious metals.

**Patentansprüche**

**1.** Herstellungsverfahren von sphärischem Vaterit, in dem

eine Partikelmischung behandelt wird, die Calcium in Form von Oxid und/oder Hydroxid in einem Reaktionsmedium enthält, welches das saure Salz eines organischen Amins enthält, so daß das Calcium in Form eines löslichen Salzes umgewandelt wird und man ein Medium erhält, dessen pH, genannt Verarbeitungs-pH, einen Wert von mehr als 10 hat und dessen Calciumgehalt zwischen 0,35 und 0,65 mol/l liegt,

$CO_2$ dem Medium zugeführt wird, das solange ausgeschüttelt wird, bis der pH des Mediums einen sogenannten Sättigungs-pH erreicht, dessen Wert sich dem 0,83fachen Verarbeitungs-pH annähert, und

$CO_2$ dem Medium bei einer Durchflußleistung unter 50 g $CO_2$/Stunde/Liter zugeführt wird, so daß sich deutlich sphärisches Vaterit bildet.

**2.** Das Verfahren gemäß Patentanspruch 1 ist dadurch gekennzeichnet, daß - nach Zuführung des Medium-pHs auf den Sättigungs-pH - $CO_2$ bei einer Durchflußleistung von weniger als 50 g $CO_2$/Stunde/Liter in das Medium eingeführt wird, so daß sich deutlich sphärisches Vaterit von bestimmter Granulometrie bildet.

**3.** Das Verfahren gemäß Patentanspruch 1 oder 2 ist dadurch gekennzeichnet, daß dem Medium $CO_2$ zugeführt wird, wenn der Calciumgehalt des Milieus zwischen 0,45 und 0,55 mol/l liegt.

**4.** Das Verfahren gemäß einem der Patentansprüche 1 bis 3 ist dadurch gekennzeichnet, daß der Calciumgehalt des Mediums so reguliert wird, daß er vor Zuführung von $CO_2$ in das Medium zwischen 0,35 und 0,65, vorzugsweise zwischen 0,45 und 0,55 mol/l, liegt.

**5.** Das Verfahren gemäß einem der Patentansprüche 1 bis 4 ist dadurch gekennzeichnet, daß $CO_2$ dem Medium zugeführt wird, wenn die Temperatur von letzterem unter 40 °C, vorzugsweise bei 20 °C, liegt.

**6.** Das Verfahren gemäß einem der vorangegangenen Patentansprüche ist dadurch gekennzeichnet, daß $CO_2$ dem Medium in drei Etappen zugeführt wird:

- Zuführung von $CO_2$ bis zum Absenken des pH auf einen sogenannten Sättigungs-pH, dessen Wert beim 0,83fachen Verarbeitungs-pH liegt,
- Zuführung von $CO_2$ bei einer Durchflußleistung von weniger als 50 g $CO_2$/Stunde/Liter bis zum Absenken des pH auf einen sogenannten Nukleations-pH, dessen Wert beim 0,8fachen Verarbeitungs-pH liegt, sowie
- Zuführung von $CO_2$ bei einer Durchflußleistung von weniger als 50 g $CO_2$/Stunde/Liter bis zum Absenken des pH auf einen sogenannten Kristallisations-pH, dessen Wert unter dem 0,75fachen Verarbeitungs-pH liegt.

Diese drei Etappen sind durch eine Zwischenetappe voneinander getrennt, in der dem Medium kein $CO_2$ zugeführt wird.

**7.** Das Verfahren gemäß einem der Patentansprüche 1 bis 6 ist dadurch gekennzeichnet, daß die Partikelmischung, die Calcium enthält, bei einer Temperatur von unter 50 °C, vorzugsweise zwischen 20 und 30 °C, verarbeitet wird.

**8.** Das Verfahren gemäß einem der vorangegangenen Patentansprüche ist dadurch gekennzeichnet, daß das Reaktionsmedium bei der ersten Etappe der Zuführung von $CO_2$ in das Medium unter einen Druck von mehr als 2 $10^5$ gesetzt wird.

**9.** Das Verfahren gemäß einem der vorangegangenen Patentansprüche ist dadurch gekennzeichnet, daß das Medium bei der ersten Etappe der Zuführung von $CO_2$ in das Medium heftig ausgeschüttelt wird (Reynolds-Zahl $N_{Re} \geqq 8000$).

**10.** Das Verfahren gemäß einem der vorangegangenen Patentansprüche ist dadurch gekennzeichnet, daß das Milieu bei der zweiten und eventuell der dritten Etappe der Zuführung von $CO_2$ in das Medium mäßig ausgeschüttelt wird ($N_{Re} \leqq 5000$).

**11.** Das Verfahren gemäß einem der vorangegangenen Patentansprüche ist dadurch gekennzeichnet, daß das saure Salz eines organischen Amins Bestandteil der allgemeinen Formel 1 ist:

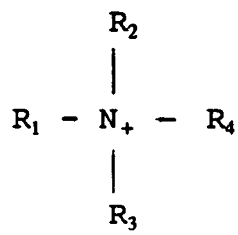

(1)

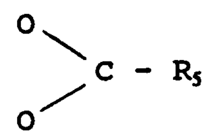

worin: mindestens $R_1$ oder $R_2$ oder $R_3$ oder $R_4$ eine Hydrogencarbonatgruppe ist, die günstigerweise von mindestens einer Hydroxylgruppe substituiert wurde.

**12.** Das Verfahren gemäß Patentanspruch 11 ist dadurch gekennzeichnet, daß sich das saure Salz des organischen Amins aus Formel 1 zusammensetzt, in der die Radikale $R_1$, $R_2$, $R_3$, $R_4$ und $R_5$ folgende Bedeutungen haben:
$R_1$ ist ein von mindestens einer Hydroxylgruppe substituiertes Hydrogencarbonat-Radikal mit 1 bis 10 Kohlenstoffatomen,
$R_2$, $R_3$ und $R_4$ sind ein Wasserstoffatom oder ein Hydrogencarbonat-Radikal, das eventuell von einer oder mehreren Hydroxylgruppen substituiert worden ist. Das Hydrogencarbonat-Radikal sollte vorzugsweise 1 bis 10 Kohlenstoffatome haben, die in $C_1$-$C_{10}$ eventuell von einer oder mehreren Hydroxyl- oder Carboxylgruppen substituiert wurden.

**13.** Das Verfahren gemäß Patentanspruch 12 ist dadurch gekennzeichnet, daß das saure Salz der Formel 1 unter der Gruppe, die das Diäthylaminacetat oder - formiat und das Monoäthylaminacetat oder -formiat enthält, ausgewählt wurde.

**14.** Das Verfahren gemäß einem der vorangegangenen Patentansprüche ist dadurch gekennzeichnet, daß die unlöslichen Stoffe nach Behandlung der Partikelmischung mit dem sauren Salz eines organischen Amins aus dem Medium entfernt wird.

**15.** Das Verfahren gemäß Patentanspruch 14 ist dadurch gekennzeichnet, daß die Calcium enthaltende Partikelmischung in einem Reaktionsmedium behandelt wird, welches das saure Salz eines organischen Amins unter Anwesenheit eines Zusatzstoffes - darunter Silicium, Aluminium, Eisen oder Eisenderivate,

Zeolithen und jeder andere poröser Träger - enthält.

**16.** Das deutlich sphärische Vaterit, das durch ein Verfahren gemäß Patentansprüchen 1 bis 15 gewonnen werden kann, ist dadurch gekennzeichnet, daß es sich in der Form einer Kugel darstellt, die zwei diametral entgegengesetzte Höhlungen sowie einen Granulometriefaktor unter 0,5 aufweist, wobei der Granulometriefaktor durch folgende Formel bestimmt wird :

$$2 \times \frac{d_{80\%} - d_{20\%}}{d_{80\%} + d_{20\%}}$$

wobei:

| | |
|---|---|
| $d_{80\%}$ | der obere Durchmesser der Partikel der Übergangsfraktion bei 80 % der Mischung ist, |
| $d_{20\%}$ | der obere Durchmesser der Partikel der Übergangsfraktion bei 20 % der Mischung ist, |
| "Übergangsfraktion von x %" | ist die Fraktion, welche die Partikel der Mischung enthält, die eine Granulometrie unter einem bestimmten Wert aufweist; sie entspricht x % des Gewichts der Mischung. |

**17.** Das Vaterit gemäß Patentanspruch 16 ist dadurch gekennzeichnet, daß es eine Granulometrie von weniger als 20 $\mu$, vorzugsweise weniger als 5 $\mu$, aufweist.

**18.** Das Vaterit gemäß Patentanspruch 17 ist dadurch gekennzeichnet, daß es eine mittlere Granulometrie von höchstens 5 $\mu$ aufweist.

**19.** Das Vaterit gemäß Patentanspruch 18 ist dadurch gekennzeichnet, daß es eine mittlere Granulometrie von weniger als oder gleich 3 $\mu$, vorzugsweise weniger als oder gleich 1 $\mu$, aufweist.

**20.** Das Vaterit gemäß einem der Patentansprüche 15 bis 19 ist dadurch gekennzeichnet, daß es mit einem Edelmetallüberzug versehen ist.

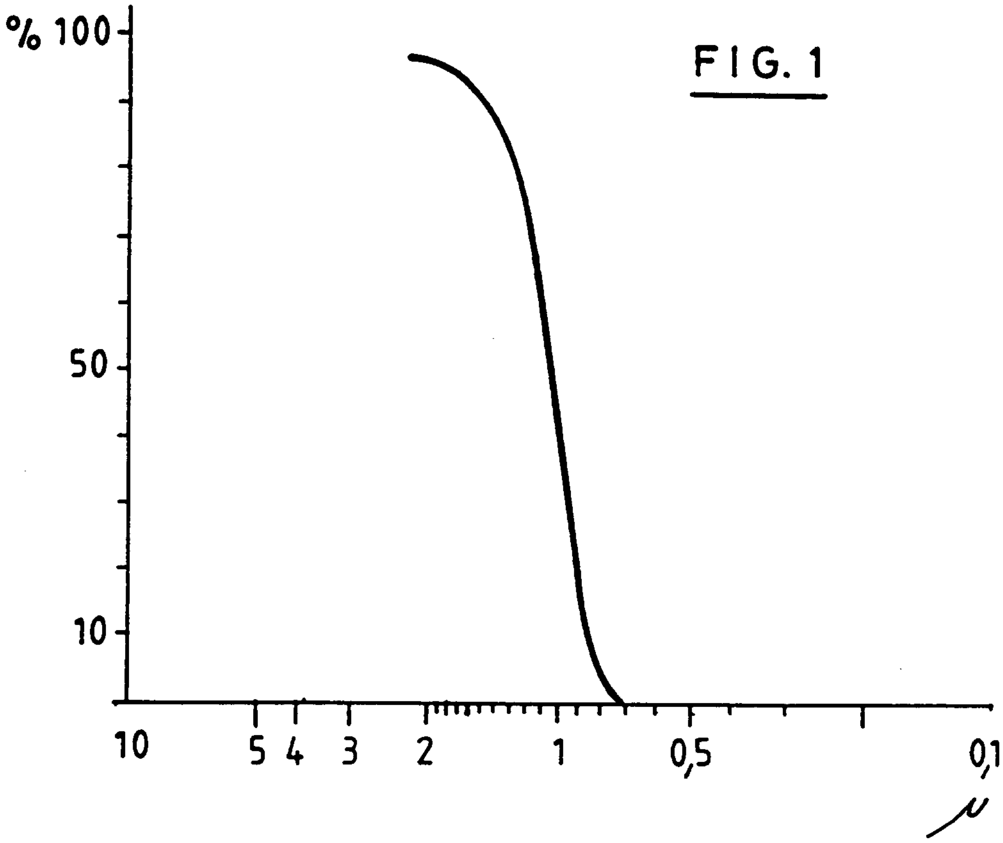


FIG. 1

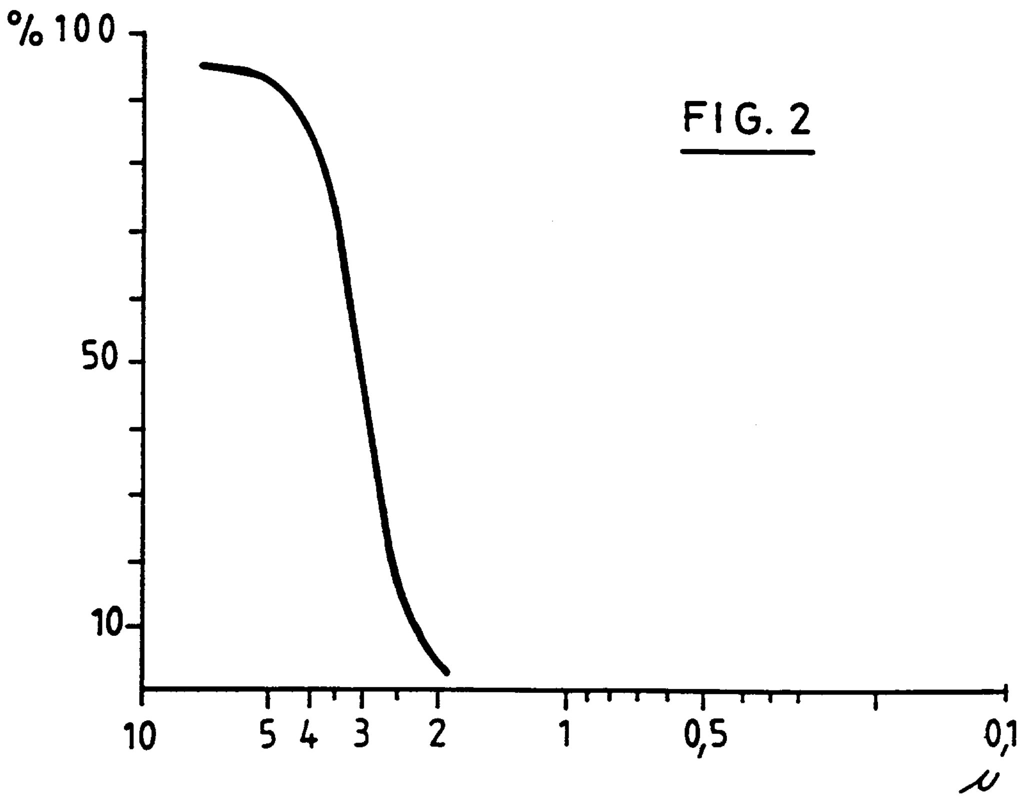


FIG. 2

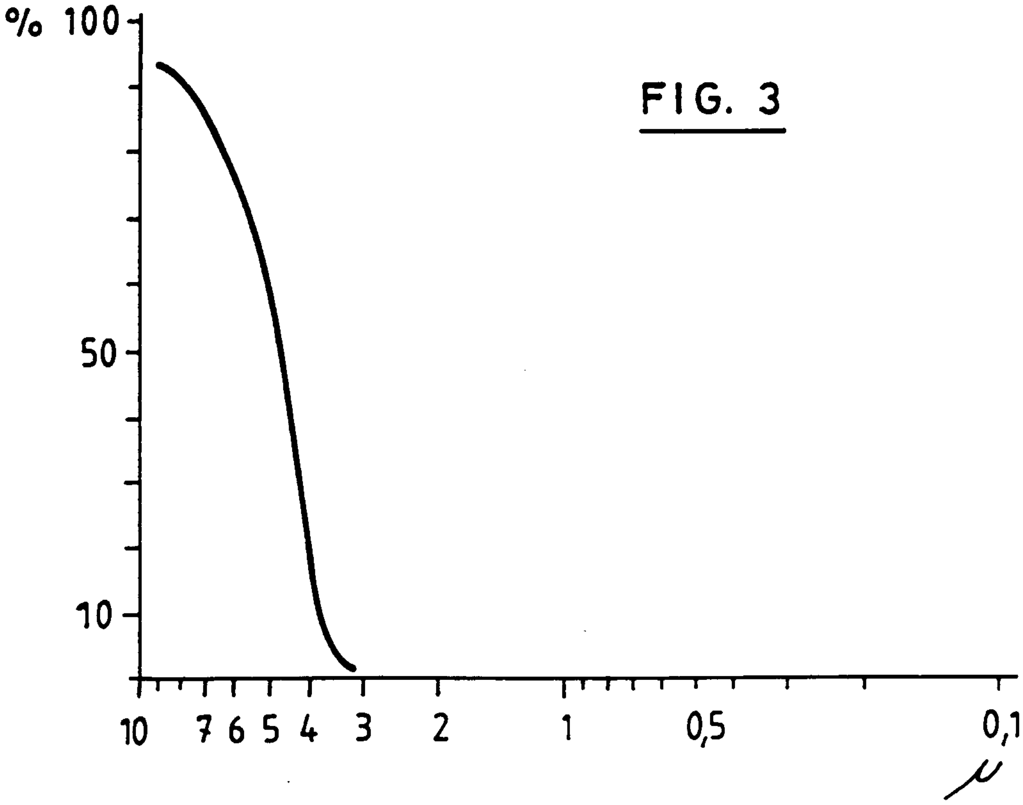


FIG. 3

FIG. 4